# EUROPEAN PATENT APPLICATION

(11) **EP 3 906 892 A1**
(43) Date of publication of application: **10.11.2021**
(21) Application number: 19907439.4
(22) Date of filing: 27.12.2019
(51) Int. Cl.: A61F 2/06, A61F 2/86, A61F 2/95, A61F 2/954

(54) **INTRAOPERATIVE STENT AND USE METHOD THEREFOR**

(30) Priority: 02.01.2019 CN 201910000771
(71) Applicant: Hangzhou Jiahe Zhongbang Biotechnology Co., Ltd, Hangzhou, Zheijiang 310023 (CN)
(72) Inventor: ZHAO, Yi Min, Beijing 100097 (CN)
(74) Representative: Lecca, Patricia S.
(86) International application number: PCT/CN2019/129104
(87) International publication number: WO 2020/140839

(57) **Abstract**

A stent, comprising a frame and a skirt (40) that covers the frame, wherein the stent comprises an axial opening (22) that penetrates the frame and the skirt (40).

## Description

### FIELD OF THE INVENTION

The present invention relates to stents and stent delivery systems.

### BACKGROUND OF THE INVENTION

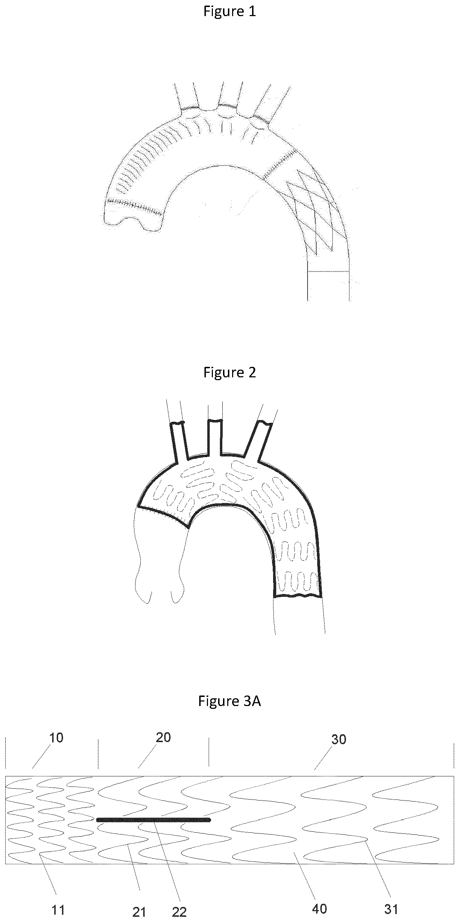

Aortic diseases seriously threaten human health and is one of the most common cardiovascular diseases. With the development of aortic endovascular treatment technology, minimally invasive interventional treatments can be implemented for both descending aortic lesions and abdominal aortic diseases that do not involve the bifurcation arteries. However, when the lesion involves the ascending aorta, the aortic arch, and the descending aorta adjacent to the aortic arch, it is still treated surgically. The traditional elephant trunk surgical procedure utilizes median sternotomy to replace the aortic arch with artificial blood vessels; however, due to the descending aorta being blocked by the heart during the median sternotomy, a piece of artificial blood vessel is usually inserted through the descending aorta incision, in order to facilitate connection for the second operation. Based on the elephant trunk procedure, improvements were made afterwards **(****Figure 1****),** where the artificial blood vessel inserted from the incision on the descending aorta is replaced by implanting a stent graft, the proximal end of the stent graft is connected with a four-branch artificial blood vessel or a straight-tube artificial blood vessel, and finally the artificial blood vessel is anastomosed with the body's own blood vessel. In comparison with the insertion of artificial blood vessels, the method of implanting the descending aorta with a stent graft reduces the operation time and can better expand the distal diseased aorta, in order to avoid possible compression and deformation of the artificial blood vessel. However, regardless of whether it is a traditional elephant trunk procedure or a modified elephant trunk procedure implanted with a stent graft, its difficulty lies in the requirement of performing freeing and anastomosis on the three branches of the aortic arch (namely innominate artery, left common carotid artery, left subclavian artery), especially for the left subclavian artery located in the deep thoracic cavity, where the freeing and the anastomosis of the artificial blood vessel is difficult to operate, in addition to the ease of damaging the recurrent laryngeal nerve and the phrenic nerve, resulting in a long operation time and a high risk.

In order to overcome the above-mentioned anastomosis on the three branches of the aortic arch, especially the left subclavian artery, some scholars invented the three-branch stent graft. This type of stent combines the stent graft implanted in the descending aorta for the modified elephant trunk procedure and the artificial blood vessel anastomosed with the body's own blood vessel into one; in addition, three small stent branches are configured on the main body of the stent, and are directly implanted in one piece to the three branches of the aortic arch, then the proximal end of the stent is anastomosed with the ascending aorta, as shown in **Figure** 2. This type of three-branch stent graft simplifies the operation and reduces possible complications caused by cardiopulmonary bypass and deep hypothermic circulatory arrest. However, the shortcomings of its poor versatility are also very apparent; due to the differences in the distance, size and shape of the three branches of the human aortic arch, the three-branch stent with fixed specifications or multiple specifications cannot adapt well to the differences between the blood vessels of the human aortic arch. As such, the three-branch stent graft provided may not be well matched with human blood vessels, and there is a risk of stenosis or blockage of the branches at a later stage, and even resulting in dislodgement of the stent.

Thus, for the treatment of aortic diseases accumulated in the aortic arch, a new type of stent graft is urgently needed, in order to achieve:
1. maj or simplification of the elephant trunk procedure to reduce its difficulty and risk
2. a universal versatility capable of adapting to the differences in the aortic arches and blood vessel branches of the human body

### SUMMARY OF THE INVENTION

In view of the above-mentioned problems, the present invention provides a stent for the treatment of aortic diseases accumulated in the aortic arch. In one embodiment, said stent comprises a frame and a skirt covering said frame, characterized in that: said stent comprises an axial opening penetrating through said frame and skirt.

The present invention further provides a stent delivery device for delivering a stent to the aortic arch. In one embodiment, said stent delivery device comprises: a main body comprising a distal end, a middle section, and a proximal end; and a binding mechanism for binding a stent to said middle section.

The present invention also provides a method for using a stent graft, comprising the following steps: (i) providing a stent graft, said stent graft comprises a frame, a skirt covering said frame, and an axial opening penetrating through said frame and skirt; (ii) placing said stent graft into a section of blood vessel of a patient, said section of blood vessel comprises at least one branch; (iii) through said axial opening, perform in-situ fenestration on said stent graft at the site corresponding to said at least one branch; (iv) implanting a stent graft along said fenestration; and (v) closing said axial opening. In one embodiment, step (ii) further comprises first binding said stent graft to a stent delivery device, and then releasing said stent graft after being placed in said section of blood vessel.

### BRIEF DESCRIPTION OF THE FIGURES

**Figure 1** is an improvement based on elephant trunk procedure, where the artificial blood vessel inserted through the descending aorta incision is changed to a stent graft, the proximal end of the stent graft is connected with a four-branch artificial blood vessel or a straight-tube artificial blood vessel, and finally the artificial blood vessel is anastomosed with the body's own blood vessel.
**Figure** 2 is a three-branch stent graft, such type of stent combines the stent graft implanted in the descending aorta for the modified elephant trunk procedure and the artificial blood vessel anastomosed with the body's own blood vessel into one; in addition, three small stent branches are configured on the main body of the stent, and are directly implanted in one piece to the three branches of the aortic arch, then the proximal end of the stent is anastomosed with the ascending aorta.
**Figure 3A** is an embodiment of the stent graft of the present invention, wherein the stent graft is tubular, comprising a first stent structural portion (10), a second stent structural portion (20) and a third stent structural portion (30), and a skirt (40) covering the surface of the stent. The first stent portion (10) comprises an internal stent frame (11); the second stent structural portion (20) comprises an internal stent frame (21) and an axial opening (22) on the skirt at a location corresponding to said second stent structural portion (20), the stent frame (21) cooperates with the opening (22) to expand the stent along the opening; the third stent portion (30) comprises an internal stent frame (31).
**Figure 3B** is another embodiment of the stent graft of the present invention, said axial opening (22) is a window-shaped opening.
**Figure 3C** is another embodiment of the stent graft of the present invention, said axial opening (22) is an arc-shaped opening.
**Figure 3D** is another embodiment of the stent graft of the present invention, the internal stent frame (21) in the second stent structural portion (20) is an open-loop frame.
**Figure 3E** is another embodiment of the stent graft of the present invention, and there is no internal stent frame in the second stent structural portion (20).
**Figure 3F** is another embodiment of the stent graft of the present invention, the internal stent frame (21) in the second stent structural portion (20) is an interlaced double-ring structure. The internal stent frame (21) is composed of a wire and a connecting tube (omitted in the figure).
**Figure 3G** is the internal stent frame (21) of **Figure** 3F. For convenience of illustration, it is divided into an inner ring (23) and an outer ring (24) of the internal stent frame. Relative movement is possible between the inner ring (23) and the outer ring (24) of the internal stent frame.

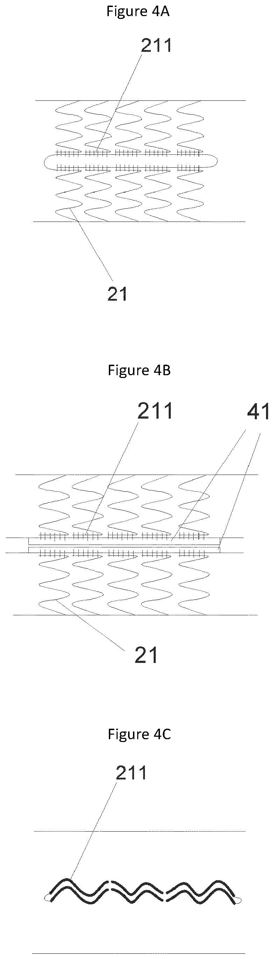

**Figure 4A** is an embodiment of the stent graft of the present invention, said axial opening (22) has a closing mechanism (211) along the opening, and in this embodiment, said closing mechanism comprises a plurality of rectilinear metal frames distributed in pairs and connected to a wave-shaped frame structure, and are connected to the skirt edge of the opening (22) by sutures; after the stent is implanted, the surgeon can fix the rectilinear metal frames (211) distributed in pairs with sutures, in order to maintain the radial rigidity of the stent structural portion (20) after closing up the opening.
**Figure 4B** is another embodiment of the stent graft of the present invention, a second skirt component (41) that extends out from the rectilinear metal frame (211) is located at where the rectilinear metal frame (211) and the skirt of the opening (22) connect, such that the surgeon can flexibly suture the second skirt component (41) with the incision on the aorta, improving the anchoring stability and sealing.
**Figure 4C** is another embodiment of the stent graft of the present invention, wherein the rectilinear metal frame is replaced by other closing mechanisms (211); this embodiment shows an interlocking matching structure.

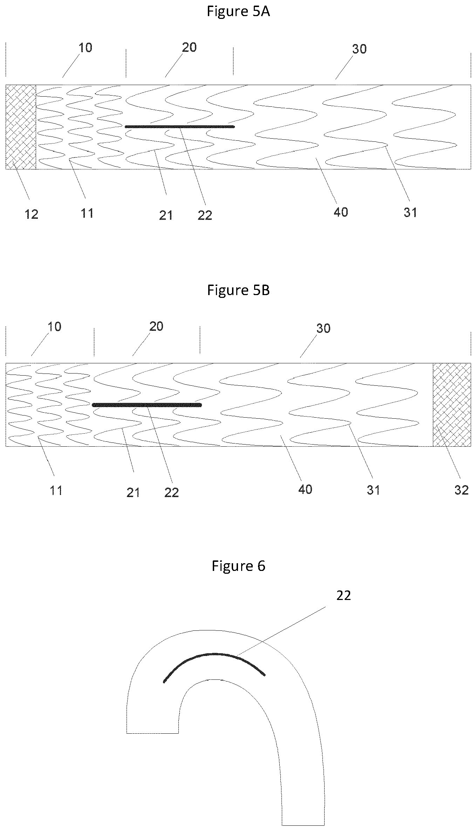

**Figure 5A** is an embodiment of the stent graft of the present invention, the first stent structural portion (10) is configured with a proximal skirt component (12) at its proximal end, and when the patient's aortic root requires simultaneous intervention, the proximal skirt component (12) can be used for the anastomosis of the ascending aorta and the aortic root, and further sutured together; the proximal skirt component (12) extends from the skirt (40) to the proximal end beyond the stent frame, and can also extend from the skirt (40) to the proximal end and flipped outward to the outer surface of the stent.
**Figure 5B** is an embodiment of the stent graft of the present invention, the third stent structural portion (30) is configured with a distal skirt component (32) at its distal end for anastomosis of the descending aorta, or for connection of artificial blood vessels when the patient's descending aorta requires another surgical intervention.
**Figure 6** is an embodiment of the stent graft of the present invention, said stent graft comprises a second stent structural portion (20) that is partially curved.

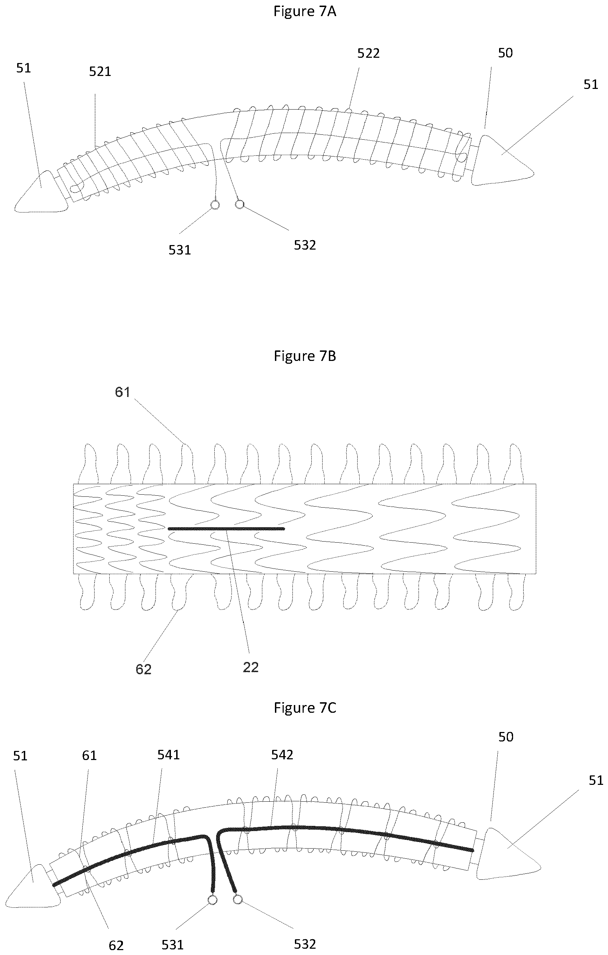

**Figure 7A** is an embodiment of the stent graft and stent delivery device of the present invention, the stent graft is loaded onto the stent delivery device (50) by tying threads, both ends of the stent delivery device (50) are designed with tapered heads (51), the tying threads (521) and (522) are divided into two sections to bind the stent graft, and the tying threads (521) and (522) each has an end connected to a pull-ring (531) and (532), when the stent graft is bound to the delivery device (50) by the tying threads, the diameter of the bound stent graft does not exceed the maximum diameter of the tapered heads; when the pull-ring (532) is pulled, the distal end of the stent is released first; when the pull-ring (531) is then pulled, the proximal part of the stent is released.
**Figure 7B** is an embodiment of a stent graft for use with a stent delivery device of the present invention, said stent graft is configured with two sets of binding threads (61) and (62), and each binding thread pairs with a corresponding binding thread.
**Figure 7C** is the stent graft and stent delivery device of the embodiment in **Figure** 7B, when the stent graft is compressed to a smaller size and assembled on the delivery device (50), each pair of binding threads (61) and (62) can first be wound around the main body of the stent, and according to the required stent segment, the free ends of the steel wires (541) and (542) pass through the binding threads (61) and (62); when the pull-ring (531) or (532) is pulled, and the steel wire (541) or (542) is withdrawn, the binding threads of the corresponding section is sprung open by the stent, thereby achieving release.
**Figure 7D** is an embodiment of the stent graft and stent delivery device of the present invention, the stent graft can be wrapped by two envelopes (71) and (72) after being compressed to the delivery device.
**Figure 7E** is the stent graft and stent delivery device of the embodiment in **Figure 7D**, after the stent graft is wrapped with the envelopes, the two ends are paired with the envelopes (71) and (72) respectively through sutures (81) and (82), and the sutures can be unwound by pulling, the ends of the sutures can be connected to pull-rings (531) and (532) respectively; when the pull-ring (531) or (532) is pulled, the envelope unfolds, and the corresponding section of the stent graft is released.
**Figures 8A to 8F** are embodiments of the method for using the present invention.

### DETAILED DESCRIPTION OF THE INVENTION

The present invention provides a stent graft with an opening, wherein said opening is configured at the curved section of the stent corresponding to the aortic arch; after the stent graft is implanted by the surgeon, the stent can flexibly undergo in-situ fenestration through the opening, and branch stents can be implanted into the three-branched blood vessels of the arch as required. Such a corresponding method reduces the number of free blood vessel anastomoses, simplifies the operation and saves operation time, and is generally capable of adapting to the differences in the blood vessels branches of the human aortic arch.

In one embodiment, said stent comprises a frame and a skirt (40) covering said frame, wherein: said stent comprises an axial opening (22) penetrating through said frame and skirt (40).

In one embodiment, said axial opening (22) is selected from rectilinear, arc-shaped or window-shaped openings.

In one embodiment, it further comprises a fenestration or a branch stent corresponding to a bifurcation position of blood vessel.

In one embodiment, the frame at said axial opening (22) portion is selected from an open-loop frame, no frame, or a frame with a double-ring structure. In another embodiment, said double-ring structure comprises an inner ring (23) and an outer ring (24), said inner ring (23) and outer ring (24) can move relative to each other.

In one embodiment, said axial opening (22) has an edge that further comprises a second skirt component (41).

In one embodiment, said frame is composed of wave-shaped or grid-like structures of equal or different spacing. In another embodiment, said material is selected from stainless steel, nickel-titanium memory alloy, cobalt-chromium alloy and the like.

In one embodiment, said stent has a distal end and a proximal end, said stent further comprises an end skirt (12, 32) located at the distal end or proximal end of said stent.

In one embodiment, said stent has a straight-tube shape, a tapered shape or a partially curved shape.

In one embodiment, said stent further comprises a closing mechanism (211) for closing said axial opening (22). In another embodiment, said closing mechanism (211) is selected from the group consisting of sutures, interlocking structure, hooking structure and rectilinear frame.

The present invention further provides a stent delivery device for delivering the stent to the aortic arch. In one embodiment, said stent delivery device comprises: a main body comprising a distal end, a middle section, and a proximal end; and a binding mechanism for binding a stent to said middle section.

In one embodiment, said distal end or proximal end is a tapered head (51).

In one embodiment, said middle section has a smaller cross-sectional size than said distal end or proximal end.

In one embodiment, said binding mechanism comprises tying ropes (521, 522), said tying ropes (521, 522) bind said stent to said middle section, and said stent is released when an end of said tying ropes (521, 522) is pulled. In one embodiment, said binding mechanism comprises two sets of binding threads (61, 62) and steel wires (541, 542), said two sets of binding threads (61, 62) are wound around said stent to bind said stent to said middle section, said steel wires (541, 542) pass through and fix said two sets of binding threads (61, 62), and when said steel wires (541, 542) are pulled, said stent is released. In one embodiment, said binding mechanism comprises envelopes (71, 72) and sutures (81, 82), said envelopes (71, 72) wrap around said stent, said sutures (81, 82) are aligned with said envelopes (71, 72) to fix said stent, and said stent is released when said sutures (81, 82) are pulled. In another embodiment, said stent delivery device further comprises pull-rings (531, 532) at an end of said tying ropes, sutures or steel wires.

In one embodiment, said stent comprises a frame and a skirt (40) covering said frame, and is characterized in that: said stent comprises an axial opening (22) penetrating through said frame and skirt (40). In another embodiment, said stent further comprises a fenestration corresponding to bifurcation position of a blood vessel.

In one embodiment, said stent further comprises a closing mechanism (211) for closing said axial opening (22).

The present invention also provides a method for using a stent graft, comprising the following steps: providing a stent graft, said stent graft comprises a frame, a skirt (40) covering said frame, and an axial opening (22) penetrating through said frame and skirt; placing said stent graft into a section of blood vessel of a patient, said section of blood vessel comprises at least one branch; through said axial opening, perform in-situ fenestration on said stent graft at the site corresponding to said at least one branch; implanting a stent graft along said fenestration; and closing said axial opening (22).

In one embodiment, said stent delivery device comprises a main body comprising a distal end, a middle section, and a proximal end; and a binding mechanism for binding a stent to said middle section; after said stent delivery device is placed in said section of blood vessel, said binding is undone, thereby releasing said stent into said section of blood vessel. In another embodiment, said binding mechanism comprises tying ropes, said tying ropes bind said stent to said middle section, and when an end of said tying ropes is pulled, said stent is released. In one embodiment, said binding mechanism comprises two sets of binding threads and steel wires, said two sets of binding threads are wound around said stent to bind said stent to said middle section, and said steel wires pass through and fix said two sets of binding threads, and when said steel wires are pulled, said stent is released. In one embodiment, the binding mechanism comprises envelopes and sutures, said envelopes wrap said stent, said sutures align with said envelopes to fix said stent, and when said sutures are pulled, said stent is released.

In one embodiment, the method for closing said axial opening (22) is selected from sutures or using an interlocking structure, a hooking structure or a rectilinear frame on the axial opening (22).

In one embodiment, said section of blood vessel comprises aortic arch.

In one embodiment, said at least one branch comprises innominate artery, left common carotid artery, or left subclavian artery.

In one embodiment, said patient is one who has established a cardiopulmonary bypass, a deep hypothermia circulatory arrest state or a moderate hypothermia circulatory arrest state.

In one embodiment, step (ii) comprises cutting said section of blood vessel axially to form a blood vessel incision. In another embodiment, step (v) further comprises any one of the following steps: (a) suturing said blood vessel incision; (b) when suturing said blood vessel incision, simultaneously sew together with the stent opening (22) that has already been closed in the previous step using sutures; (c) said stent further comprises a second skirt component (41), when suturing said blood vessel incision, the second skirt component (41) and said blood vessel incision are sutured together; or (d) suture and fix said stent with said blood vessel incision.

### EXAMPLE 1

In one embodiment, the present invention provides a stent, comprising a frame and a skirt (40) covering said frame, characterized in that: said stent comprises an axial opening (22) that penetrates said frame and skirt (40). In one embodiment, said stent is shown in Figure 3A, where the stent graft is tubular, comprising a first stent structural portion (10), a second stent structural portion (20) and a third stent structural portion (30), and a skirt (40) covering the surface of the stent. The first stent portion (10) comprises an internal stent frame (11); the second stent structural portion (20) comprises an internal stent frame (21) and an axial opening (22) on the skirt at a location corresponding to said second stent structural portion (20), the stent frame (21) cooperates with the opening (22) to expand the stent along the opening; the third stent portion (30) comprises an internal stent frame (31). The surgeon can cut the aortic arch along the axial direction of the vessel to form an incision, the stent graft can be loaded onto the delivery system through tying with threads or wrapping, and can enter the aortic arch and adjacent locations through the aortic arch incision; after the release is complete, the surgeon can perform in-situ fenestration of the stent graft for the three blood vessel branches of the arch through the opening (22), and then place the stent graft; after completing the three-branch artery, close the stent opening (22) and the aortic incision with sutures, and the operation is complete.

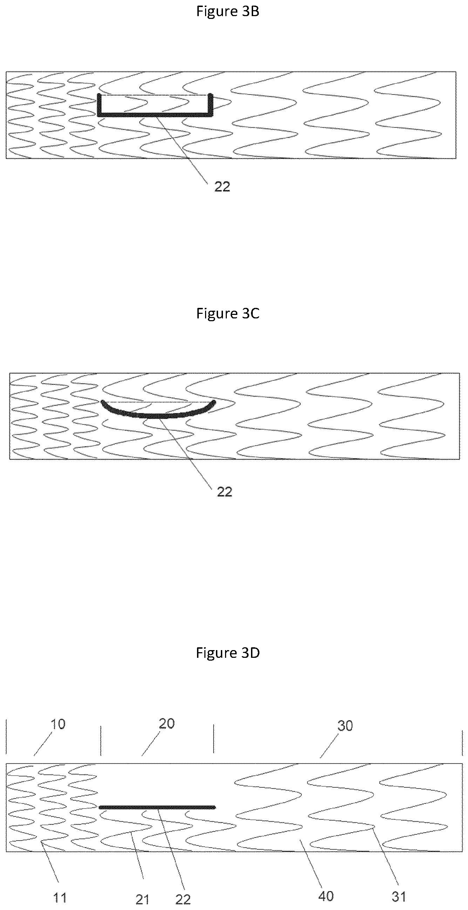

As described above, the shape of the opening (22) of said stent graft can be rectilinear as shown in Figure 3A, or any opening shape that is conducive to opening, such as a window-shaped opening or an arc-shaped opening, as shown in **Figures 3B** and **3C****.**

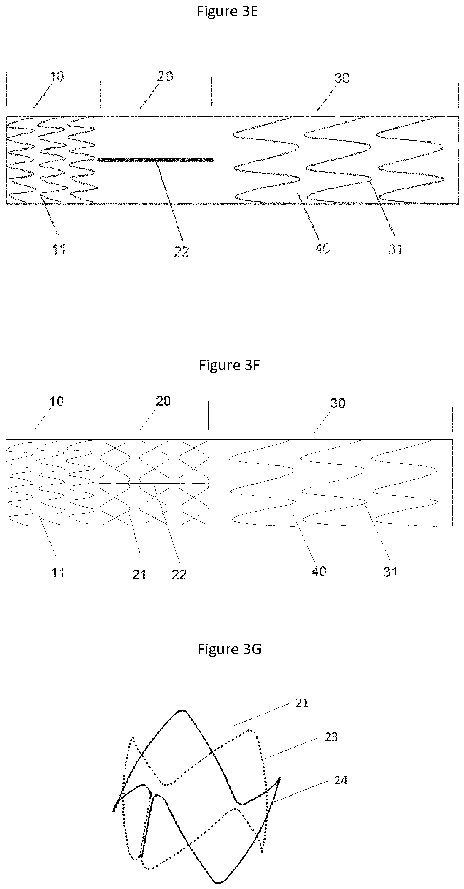

As described above, the internal stent frame (21) in the second stent structural portion (20) of said stent graft may be an open-loop frame as shown in **Figure 3D**; or as shown in **Figure 3E**, the second stent structural portion (20) does not have an internal stent frame; or as shown in **Figure 3F** and **Figure 3G**, an interlaced double-ring structure.

In the stent graft as described above, the stent frame (11, 21, 31) can be constructed with a plurality of identical or different wave-shaped or grid-like metal wire frames, can be constructed by weaving metal wires according to equal or different grid spacing, or can be constructed by laser-cutting, expanding and shaping a grid-like metal frame. The metal raw materials can be stainless steel wire, nickel-titanium memory alloy wire, cobalt-chromium alloy wire, nickel-titanium tubes and the like.

In the stent graft as described above, the skirt (40) is connected to the stent frame (11, 21, 31) through sutures or an integrated film, forming the stent graft. The skirt material can be PET woven fabric, PTFE woven fabric, silicone rubber, and other biocompatible film-like materials. The skirt (40) is located on the outside and/or inside of the stent frame (11, 21, 31). The skirt (40) can be designed as a flat surface, or as an undulating wavy surface.

### EXAMPLE 2

In one embodiment, the stent provided in the present invention further comprises a closing mechanism (211) for closing said axial opening (22). In one embodiment, said stent graft is shown in **Figure 4A****,** where the stent frame (21) of the second stent structural portion (20) has a plurality of rectilinear metal frames (211) distributed in pairs along the opening (22) and connected to the wave-shaped frame structure, and is connected to the skirt edge of the opening (22) through sutures. After the stent is implanted, the surgeon can fix the rectilinear metal frames (211) distributed in pairs with sutures, for maintaining the radial rigidity of the stent structural portion (20) after closing the opening. In another embodiment, on the basis of the above content, a second skirt component (41) that extends out from the rectilinear metal frame (211) can be located at where the rectilinear metal frame (211) and the skirt of the opening (22) connect, as shown in **Figure 4B****,** such that the surgeon can flexibly suture the second skirt component (41) with the incision on the aorta, improving the anchoring stability and sealing. In one embodiment, in addition to the rectilinear metal frame, the closing mechanism (211) can also be changed to an interlocking matching structure, or a hooking structure as shown in **Figure 4C****,** all of which can be associated with this technical field on such basis, and will not deviate from the scope of the present invention.

### EXAMPLE 3

In one embodiment, the stent provided in the present invention further comprises a skirt component (12, 32) located at the distal end or proximal end of said stent. In one embodiment, the stent is shown in **Figure 5A****,** where the first stent structural portion (10) can also be configured with a proximal skirt component (12) at the proximal end, when the patient's aortic root requires simultaneous intervention, the proximal skirt component (12) can be used for anastomosis of the ascending aorta and the aortic root, and further sutured together. The proximal skirt component (12) can extend from the skirt (40) to the proximal end beyond the stent frame, or it can extend from the skirt (40) to the proximal end and flipped outward to the outer surface of the stent.

In the stent graft as described above, as shown in **Figure 5B****,** the distal end of the third stent structural portion (30) can be configured with a distal skirt component (32) for anastomosis of the descending aorta, or for connection of artificial blood vessels when the patient's descending aorta requires another surgical intervention.

In one embodiment, a stent can be configured with both proximal skirt component (12) and distal skirt component (32), and their material can be PET woven fabric or PTFE woven fabric and the like.

In one embodiment, the distal skirt (32) is not sutured when the stent is implanted, but instead provides for easy surgical suturing in the patient's possible future operations.

In one embodiment, the situations of suturing the proximal skirt (12) is selected from: 1) when the patient's aortic root requires surgical intervention at the same time and is incised, the proximal skirt (12) can be sutured together with the two sections of proximally anastomosed blood vessels; 2) to ensure that there is no endoleak at the proximal end of the stent (proximal endoleak: the proximal end is not tightly fitted, blood enters the space between the stent and the blood vessel wall), the surgeon cuts open the proximal blood vessel, and then the proximal skirt (12) is sutured together with the two sections of anastomosed blood vessels.

In one embodiment, the proximal skirt (12) may be sutured together with the blood vessel any time after the stent is placed in the blood vessel.

### EXAMPLE 4

In one embodiment, the stent provided in the present invention may have different shapes. In one embodiment, the tubular feature of said stent graft may be a constant diameter tube. In another embodiment, said stent graft may have a tapered shape with a gradually changing diameter, that is, diameter D1 of the first stent structural portion (10) is greater than diameter D2 of the second stent structural portion (20), and diameter D2 of the second stent structural portion (20) is greater than diameter D3 of the third stent structural portion (30). In one embodiment, the overall tubular shape of said stent graft may be a straight tubular shape. In one embodiment, said stent graft is shown in **Figure 6**, which may be partially curved, and the position of the partially curved section may be the second stent structural portion (20).

### EXAMPLE 5

The present invention further provides a stent delivery device suitable for delivering the stent of the present invention. In one embodiment, the stent delivery device of the present invention comprises: a main body comprising a distal end, a middle section, and a proximal end; and a binding mechanism for binding a stent to said middle section. In one embodiment, said binding mechanism comprises tying ropes, said tying ropes bind said stent to said middle section, and when an end of said tying ropes is pulled, said stent is released. In another embodiment, said stent graft, as shown in **Figure 7A**, can be loaded onto the delivery device (50) by method of tying threads. Both ends of the delivery device (50) are designed with tapered heads (51), the tying threads (521, 522) are divided into two sections to bind the stent graft, and the thread ends of the tying threads (521, 522) are each connected to a pull-ring (531, 532), when the stent graft is bound to the delivery device (50) by tying threads, its bound diameter does not exceed the maximum diameter of the tapered head (51). When the pull-ring (532) is pulled, the distal end of the stent is released first; when the pull-ring (531) is then pulled, the proximal part of the stent is released. The main body of the delivery device (50) is curve-shaped or arc-shaped to match the anatomical characteristics of the aortic arch; optionally, the main body of the delivery device (50) can also be a main body that can be freely shaped, and depending on the requirements, the surgeon adjusts the curved shape of the delivery device (50) after loading the stent graft, in order to better adapt to the anatomy of the aortic arch. Optionally, a penetrating cavity can also be configured inside the delivery device (50) for in-situ adjustment of surgical procedures and for the deployment of guide wires.

### EXAMPLE 6

As another embodiment of the binding and release method, said binding mechanism of the present invention comprises two sets of binding threads and steel wires, said two sets of binding threads are wound around said stent to bind said stent to said middle section, said steel wires pass through and fix said two sets of binding threads, and when said steel wires are pulled, said stent is released. In one embodiment, said stent graft can be configured with two sets of binding threads (61, 62), and each binding thread pairs with a corresponding binding thread, as shown in Figure 7B; when the stent graft is compressed to a smaller size and assembled on the delivery device (50), each pair of binding threads (61, 62) can first be wound around the main body of the stent, and according to the required stent segment, the free ends of the steel wires (541, 542) pass through the binding threads (61, 62), and when the pull-ring (531, 532) is pulled, and the steel wire (541, 542) is withdrawn, the binding threads (61, 62) of the corresponding section is sprung open by the stent to achieve release, as shown in **Figure 7C**. The binding threads (61, 62) can be general surgical sutures, ePTFE or PTFE threads, or bioresorbable sutures.

### EXAMPLE 7

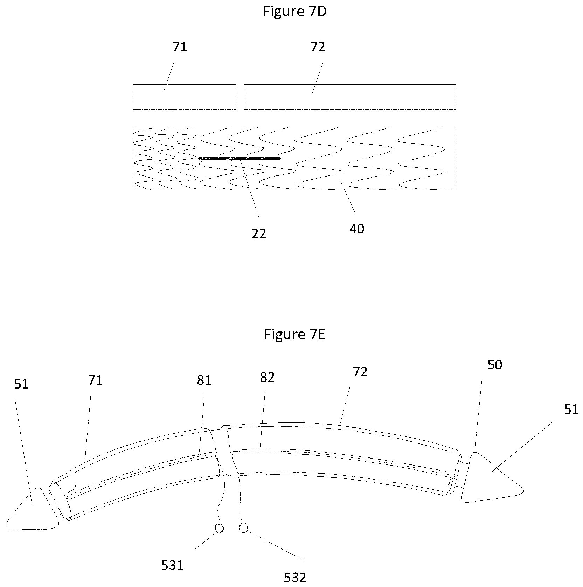

As another embodiment of the binding and release method, said binding mechanism of the present invention comprises envelopes and sutures, said envelopes wrap the stent, said sutures align with said envelopes to fix said stent, and when said sutures are pulled, said stent is released. In one embodiment, as shown in **Figure 7D** and **Figure 7E**, the stent graft can be wrapped by two envelopes (71, 72) after being compressed to the delivery device, after the stent graft is wrapped with the envelope, the two ends are paired with the envelopes (71, 72) respectively through sutures (81, 82), and the sutures (81, 82) can be unwound by pulling, the ends of the sutures can be connected to the pull-rings (531, 532) respectively; when the pull-rings (531, 532) are pulled, the envelope unfolds, and the corresponding section of the stent graft is released. The material of the envelopes can be a PET skirt or PTFE skirt, or other conventional macromolecular woven fabric materials; the sutures (81, 82) can also be metal wires.

### EXAMPLE 8

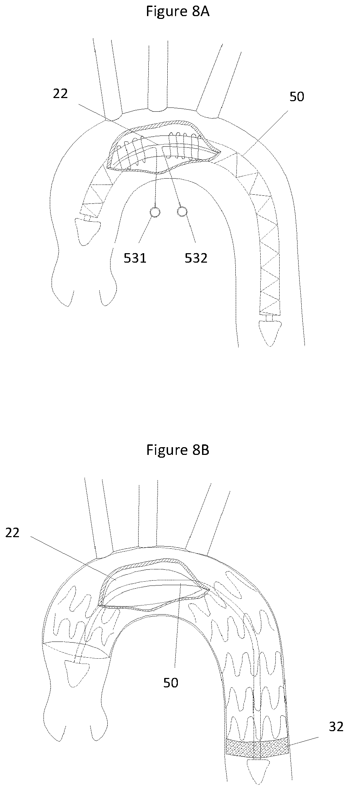

The present invention further provides methods of using the stent graft. In one embodiment, the method of using said stent graft is described as follows: under a state of cardiopulmonary bypass, deep hypothermic circulatory arrest or moderate hypothermic circulatory arrest, the aortic arch is incised along the axial direction, and the delivery device (50) loaded with the stent graft is inserted from the aortic arch incision, inserted deeply towards the descending aorta end first, and then place the entire delivery device (50) into the blood vessel; according to the position of the stent graft opening (22), its position is adjusted appropriately, as shown in **Figure 8A**.

First pull the pull-ring (532) to release the distal portion of the stent graft, after the thread pulling is complete, the stent graft is released at the descending aorta section and at positions adjacent to the arch; then pull the pull-ring (531), and the stent is expanded at the remaining arch section and ascending aorta section. Opening up the stent graft opening (22) allows us to see the delivery device (50), as shown in **Figure 8B**; take the delivery device out from the stent graft opening (22).

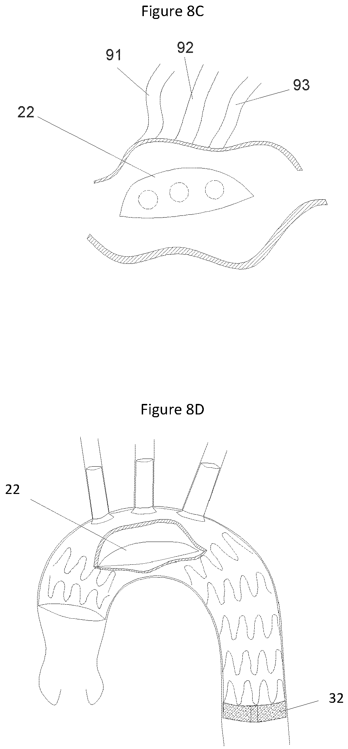

Through the stent graft opening (22), with the three branches of the aortic arch as the reconstruction target, in-situ fenestration of the stent graft is performed. The three fenestrations correspond to the innominate artery (91), the left common carotid artery (92) and the left subclavian artery (93) respectively; as shown in **Figure 8C**. In one embodiment, said in-situ fenestration comprises laser fenestration and other fenestration methods suitable for clinical use. In one embodiment, in order to avoid interference between the fenestration position and the internal stent frame (21) in the second stent structural portion (20), the internal stent frame (21) is an open-loop frame as shown in **Figure 3D**; in another embodiment, in order to avoid interference between the fenestration position and the internal stent frame (21) in the second stent structural portion (20), there is no internal stent frame in the second stent structural portion (20) (as shown in **Figure 3E**); in another embodiment, the internal stent frame (21) in the second stent structural portion (20) is the interlaced double-ring structure in **Figure 3F** and **Figure 3G****,** when the fenestration position interferes with the internal stent frame (21), the relative positional relationship between the inner ring (23) and the outer ring (24) of the internal stent frame can be adjusted to avoid interference.

Through the stent graft opening (22), the innominate artery (91), the left common carotid artery (92), and the left subclavian artery (93) were reconstructed by implanting stent grafts along the three fenestrations respectively; as shown in **Figure 8D**. In one embodiment, the surgeon may further connect the stent graft implanted in the blood vessel branch to the fenestration position of said stent graft through suturing or other means, so as to improve the stability of the stent graft in the blood vessel branch and/or the sealing between the stent graft in the blood vessel branch and the arch fenestration stent.

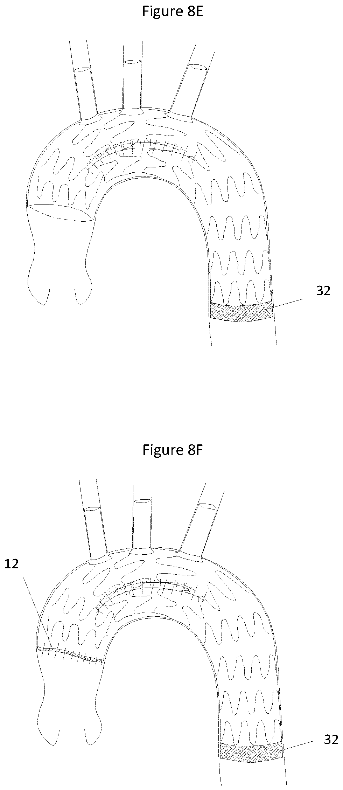

The stent graft opening (22) is sutured and closed, and the aortic arch incision is sutured to complete the implantation, as shown in **Figure 8E****.** When the stent graft opening (22) is configured with a second skirt component (41), the second skirt component (41) and the aorta wall of the aortic arch incision can be sutured together after the stent graft opening (22) is sutured and closed. Then, after deairing, circulation is restored and temperature is restored.

When the surgeon deems necessary according to the situation, or the aortic root requires replacement intervention, surgery on the root section or incision treatment can be performed first, and then the stent graft with a proximal skirt component (12) is implanted. The proximal skirt component (12) can be sutured together with the anastomosed ascending aorta, or sutured together with the prosthetic root section and the ascending aorta; as shown in **Figure** 8F.

The unique opening designs and methods of using the stent graft can simplify the elephant trunk procedure for the surgeon, reducing the operation time and surgical risk; at the same time, the method of reconstructing the three-branch artery with the in-situ fenestration in cooperation with the stent graft opening greatly improves the adaptability of the apparatus to the differences in the blood vessel branches of the arch, and thus possesses good clinical application value.

Those of ordinary skill in the art shall understand that the content of this specification is merely a conceptual demonstration, and does not limit the scope of protection of the present invention, which is defined by the following claims.

## Claims

1. A stent, comprising a frame and a skirt (40) covering said frame, wherein:
said stent comprises an axial opening (22) penetrating through said frame and skirt (40).

2. The stent of claim 1, wherein said axial opening (22) is selected from rectilinear, arc-shaped or window-shaped openings.

3. The stent of claim 1, further comprising a fenestration or a branch stent corresponding to a bifurcation position of blood vessel.

4. The stent of claim 1, wherein the frame at said axial opening (22) portion is selected from an open-loop frame, no frame, or a frame with a double-ring structure.

5. The stent of claim 4, wherein said double-ring structure comprises an inner ring (23) and an outer ring (24), said inner ring (23) and outer ring (24) can move relative to each other.

6. The stent of claim 1, wherein said axial opening (22) has an edge that further comprises a second skirt component (41).

7. The stent of claim 1, wherein said frame is composed of wave-shaped or grid-like structures of equal or different spacing.

8. The stent of claim 7, wherein said material is selected from stainless steel, nickel-titanium memory alloy or cobalt-chromium alloy.

9. The stent of claim 1, wherein said stent has a distal end and a proximal end, said stent further comprises an end skirt (12, 32) located at the distal end or proximal end of said stent.

10. The stent of claim 1, wherein said stent has a straight-tube shape, a tapered shape or a partially curved shape.

11. The stent of any one of claims 1-10, further comprising a closing mechanism (211) for closing said axial opening (22).

12. The stent of claim 11, wherein said closing mechanism (211) is selected from the group consisting of sutures, interlocking structure, hooking structure and rectilinear frame.

13. A stent delivery device for delivering the stent of claim 1, comprising:
a main body comprising a distal end, a middle section, and a proximal end; and
a binding mechanism for binding said stent to said middle section.

14. The stent delivery device of claim 13, wherein said distal end or proximal end is a tapered head (51).

15. The stent delivery device of claim 13, wherein said middle section has a smaller cross-sectional size than said distal end or proximal end.

16. The stent delivery device of claim 13, wherein said binding mechanism comprises tying ropes (521, 522), said tying ropes (521, 522) bind said stent to said middle section, and said stent is released when an end of said tying ropes (521, 522) is pulled.

17. The stent delivery device of claim 13, wherein said binding mechanism comprises two sets of binding threads (61, 62) and steel wires (541, 542), said two sets of binding threads (61, 62) are wound around said stent to bind said stent to said middle section, said steel wires (541, 542) pass through and fix said two sets of binding threads (61, 62), and when said steel wires (541, 542) are pulled, said stent is released.

18. The stent delivery device of claim 13, wherein said binding mechanism comprises envelopes (71, 72) and sutures (81, 82), said envelopes (71, 72) wrap around said stent, said sutures (81, 82) are aligned with said envelopes (71, 72) to fix said stent, and said stent is released when said sutures (81, 82) are pulled.

19. The stent delivery device of any one of claims 16-18, further comprising pull-rings (531, 532) at an end of said tying ropes, sutures or steel wires .
